# EUROPEAN PATENT SPECIFICATION

(11) **EP 3 037 108 B1**
(45) Date of publication and mention of the grant of the patent: **05.04.2017**
(21) Application number: 15190993.4
(22) Date of filing: 22.10.2015
(51) Int. Cl.: A61L 2/18

(54) **SYSTEM FOR THE FAST CONNECTION OF A PIPE TO AN OUTLET AND THE AUTOMATIC CLOSING OF THE OUTLET IN THE ABSENCE OF THE PIPE**
SYSTEM ZUR SCHNELLEN VERBINDUNG EINES ROHRES AN EINEN AUSLASS UND AUTOMATISCHES SCHLIESSEN DES AUSLASSES IN ABWESENHEIT DES ROHRES
SYSTÈME POUR LE RACCORDEMENT RAPIDE D'UN TUYAU À UN ORIFICE DE SORTIE ET FERMETURE AUTOMATIQUE DE LA SORTIE EN L'ABSENCE DU TUYAU

(30) Priority: 12.11.2014 IT MI20141953
(43) Date of publication of application: 29.06.2016
(73) Proprietor: Bonferraro S.p.A., 37060 Bonferraro (VR) (IT)
(72) Inventor: GOBBI, Ezio, 46037 Roncoferraro MN (IT); TOSI, Mauro, 37051 Bovolone VR (IT); BENFATTI, Oscar, 37063 Isola della Scala VR (IT)
(74) Representative: Concone, Emanuele

(56) References cited:
- EP-A1- 2 898 901
- WO-A1-00/35529
- DE-A1-102013 014 254
- US-A1- 2013 186 428
- US-B1- 6 391 258

## Description

The present invention relates to systems for the fast connection of pipes and the automatic closing of outlets, and in particular to such a system applied to the trolley of an apparatus for the disinfection of medical items such as surgical instruments, cannulas for laparoscopy and the like. In the following specific reference will be made to this application of the present system, but it is clear that what is said can also be applied in other fields which have similar requirements of speed of connection of a pipe to a fluid feed outlet and of automatic closing of said outlet in the absence of the pipe.

It is known that a trolley for the washing and disinfection of medical items essentially consists in a plurality of horizontal shelves provided with bottom sprinklers, each sprinkler being fed through a pipe which branches off from a single central manifold which extends vertically along the whole height of the trolley. For the washing of hollow items, such as typically the cannulas for laparoscopy, there is used a rack comprising a wash duct shaped to accommodate said hollow items and equipped with outlets which spray the washing liquid inside the hollow items (e.g. US 2013/186428).

Therefore, the feed manifold of the trolley is also provided with at least one outlet to which a flexible pipe can be connected for the connection to the wash duct of the rack possibly arranged on a corresponding shelf of the trolley. In the absence of the rack such an outlet must remain closed to avoid both a waste of water and a pressure drop in the manifold which would negatively affect the cleaning efficiency of the sprinklers.

In prior art trolleys the outlet is closed by a simple plug or by an automatic valve disposed within the outlet and actuated open by the pipe itself at the time of its insertion into the outlet. The plug solution is very simple and cheap but scarcely reliable, because the operator may forget to apply it after detaching the rack pipe or the plug may be applied badly and the plug can come away during washing due to the pressure in the manifold, and furthermore it may be lost when it is removed to insert the rack pipe.

The automatic valve prevents forgetfulness and remains on the carriage, but it is a rather complex and costly solution in addition to being not entirely reliable. In fact the valve mechanism may be deteriorated or damaged from the continuous exposure to the high temperatures and pressures that occur during the washing cycle, so that the valve is partially ineffective, without the operator noticing it given the internal position of the valve. Consequently, the pressure in the manifold during operation of the machine with the outlet closed might be lower than the nominal washing pressure, thus causing a decrease in the washing effectiveness.

The object of the present invention is therefore to provide a system that overcomes the above-described limitations of prior art sprinklers. This object is achieved by means of a system comprising a bushing mounted externally on the outlet and provided with a slot, a gate mounted on the manifold and mobile between a position closing said bushing through introduction into said slot and a position opening the bushing, and a terminal suitable to be mounted at the end of the flexible pipe and provided with a peripheral groove suitable to receive said gate as well as sized and shaped for internal or external coupling with the bushing. Other advantageous features are disclosed in the dependent claims.

The main advantage of the system according to the present invention is to ensure a high reliability combined with a low cost and an automatic operation.

A further advantage of this system stems from the fact that its components have a higher strength and are easily verifiable by the operator, thanks to the arrangement on the outside of the outlet.

These and other advantages and characteristics of the system according to the present invention will become apparent to those skilled in the art from the following detailed description of two embodiments thereof with reference to the accompanying drawings in which:
Fig.1 is a perspective top view of a trolley comprising a first embodiment of the system according to the invention, in the closing position;
Fig.2 is a view similar to the previous one with the system in the opening position and a rack pipe connected to the feed outlet;
Fig.3 is an enlarged exploded view of the components of said system, including a variant of the pipe terminal;
Fig.4 is a vertical sectional view along a midplane of the system in the closing position as in Fig.1;
Fig.5 is a view similar to the previous one with the system in the opening position and with the pipe connected by using the above-mentioned variant of the terminal;
Fig.6 is an enlarged exploded view of the components of a second embodiment of the system; and
Fig.7 is a perspective view of the second embodiment in the assembled condition.

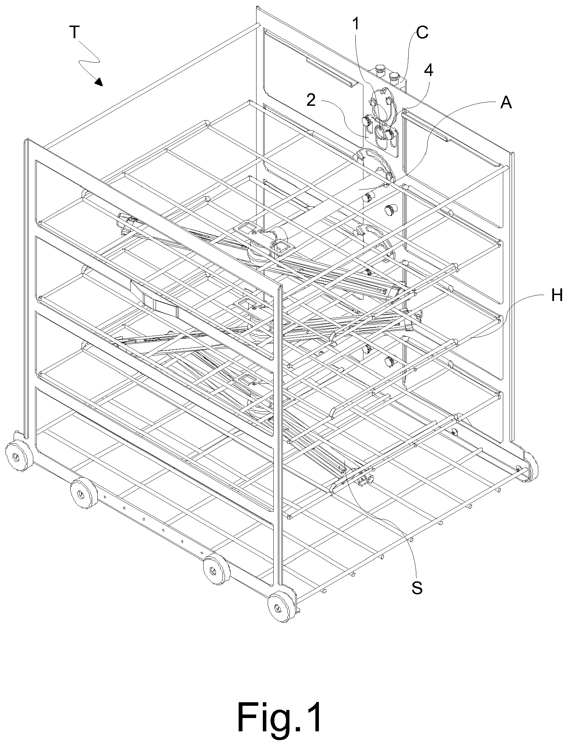

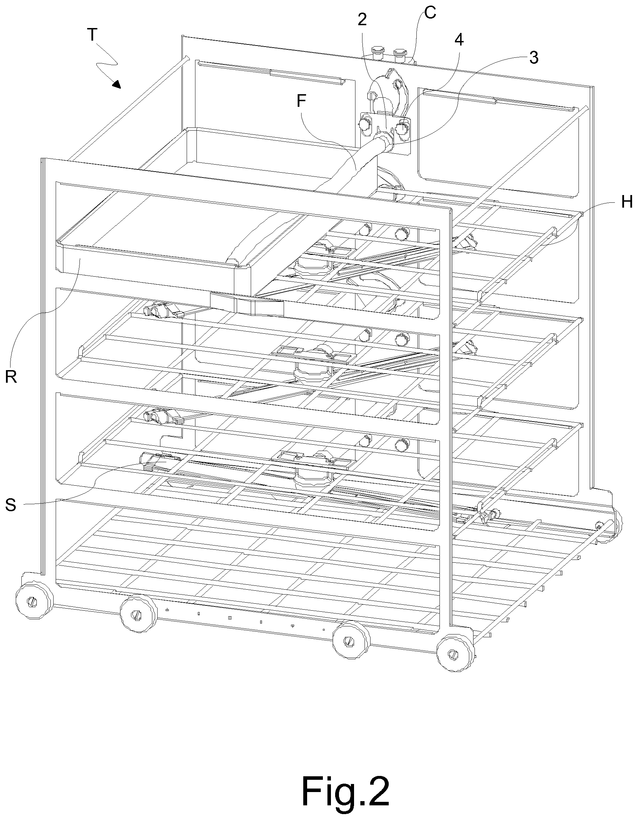

Referring to figures 1 and 2, there is seen that a trolley T for the washing and disinfection of medical items essentially consists in a plurality of horizontal shelves H provided with bottom sprinklers S, each sprinkler S being fed through a pipe A which branches from a single central manifold C which extends vertically along the whole height of trolley T. For the washing of hollow articles there is used a rack R arranged on one of shelves H and comprising a wash duct (not shown) connected to manifold C through a flexible pipe F that connects to a corresponding outlet formed in manifold C.

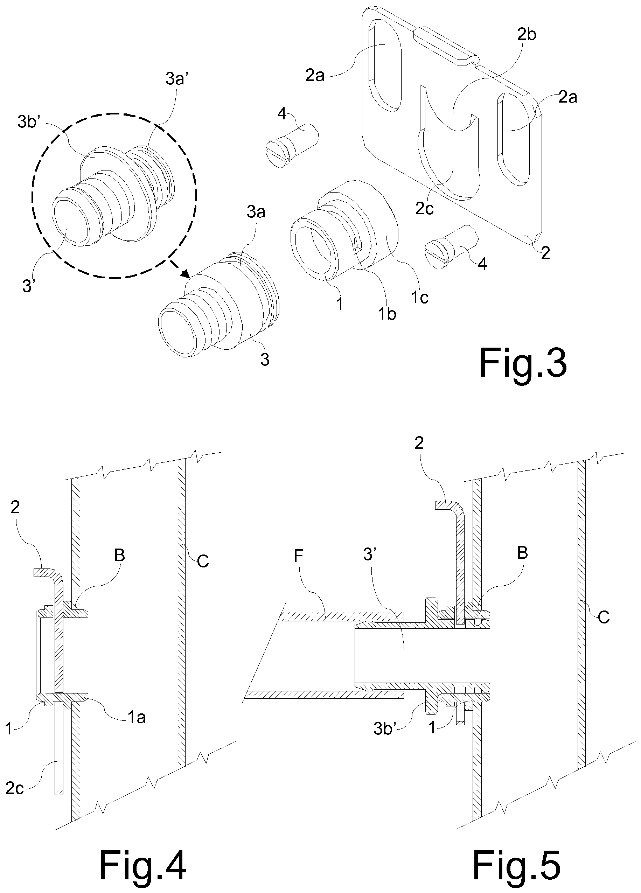

The system according to the present invention is now illustrated with reference also to figures 3 to 5, which illustrate in greater detail the above-mentioned components of the system, i.e. a bushing 1, a gate 2 and a terminal 3/3' of pipe F.

More specifically, bushing 1 is mounted on an outlet B by introducing therein a corresponding rear portion 1a (not visible in Fig.3) so as to protrude externally, and is provided with an external slot 1b which extends on the upper half of its perimeter. Gate 2 is mounted on manifold C by means of a pair of screws 4 which engage corresponding elongated vertical eyelets 2a, so that gate 2 is vertically slidable. In this way, gate 2 is movable between position closing bushing 1, through introduction into slot 1b of its corresponding portion 2b (Figs. 1 and 4), and a position opening bushing 1 (Figs.2 and 5) in which portion 2b of gate 2 is lifted to allow the connection of the flexible pipe F.

In particular, terminal 3/3' arranged at the end of pipe F can be sized and shaped to couple with bushing 1 either externally (Fig.2, terminal 3) or internally (Fig.5, terminal 3'), and is however provided with a peripheral groove 3a/3a' suitable to receive the aforesaid portion 2b so as to lock pipe F on bushing 1. Note that in the first case terminal 3 is fitted on bushing 1 until it abuts on the front end of an enlarged portion 1 c thereof, while in the second case terminal 3' is also provided with a flange 3b' that abuts on the front end of bushing 1 so as to define the depth of introduction and thus the correct position of groove 3a' in correspondence of slot 1b when gate 2 is lowered to lock pipe F.

The locking of bushing 1 into outlet B is achieved inwards through the rear portion 1a of reduced size with respect to the adjacent portion 1c, while outwards it is gate 2 itself that prevents the displacement of bushing 1 by means of a central opening 2c substantially shaped like an inverted keyhole. In other words, the upper portion of opening 2c, which is the one affected by the travel of gate 2 during its operation, has a width smaller than portion 1c of bushing 1 and therefore does not allow it to go through, while the lower portion of opening 2c is wide enough to allow the passage of bushing 1 in the assembly phase before gate 2 is fastened with screws 4.

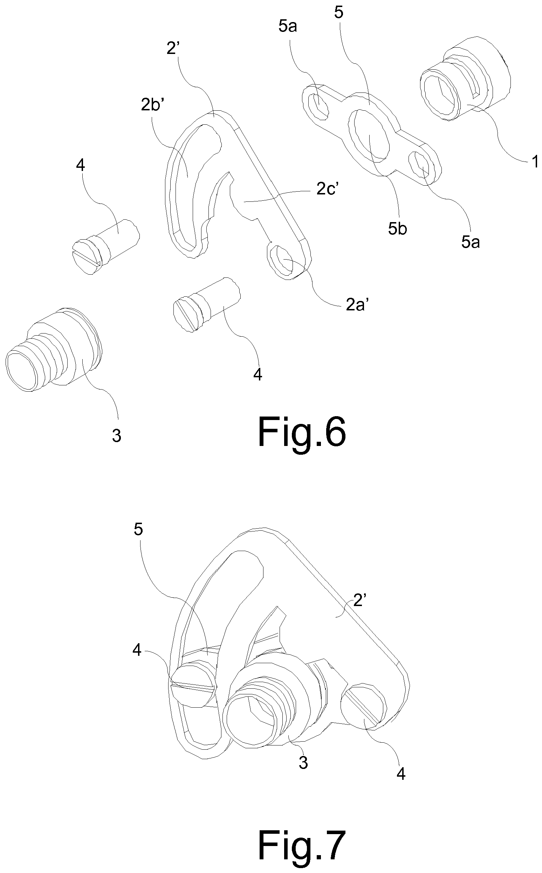

Finally, referring to figures 6 and 7, there is illustrated a second embodiment of the present system that is different from the first embodiment with respect to the gate, while bushing 1, terminal 3 and the fixing screws 4 remain unchanged. In practice, this second embodiment provides for a rotating gate 2' instead of the sliding gate 2 of the first embodiment.

More specifically, gate 2' presents on one side a hole 2a' for a first screw 4 and on the opposite side an arcuate slot 2b', for the second screw 4, having a radius and a center of curvature that allow the rotation of gate 2' around hole 2a'. Moreover, a central portion 2c', corresponding to portion 2b of the sliding gate 2, extends downwards for the introduction into slot 1b and/or in groove 3a/3a'.

Note that because of its different shape, the rotary gate 2' is not able to perform also the function of outward stop for bushing 1 as did the sliding gate 2, therefore this function is performed by a locking bracket 5. Said bracket 5 has two lateral holes 5a for screws 4 and a central hole 5b sized to block portion 1c of bushing 1, and could also be used in an embodiment with a sliding gate if the latter had a shape unsuitable to retain bushing 1.

Furthermore, although in figures 6 and 7 there is not represented variant 3' of the terminal of pipe F, it is clear that also this type of terminal may be used in the second embodiment.

In both embodiments the movement of gate 2/2' from the closing position to the opening position requires the intervention of an operator, while the reverse movement takes place automatically by gravity or preferably with the aid of a return spring (not shown in the figures).

It is clear that the above-described and illustrated embodiments of the system according to the invention are just examples susceptible of various modifications. In particular, the exact shape of components 1, 2/2', 3/3' and possibly 5 may vary somewhat depending on specific production needs as long as the general structure shown above is maintained.

For example, although components 1 and 3/3' are illustrated as having circular cross-section, nothing prevents to realize them with sections of other shapes, in which case also the shape and size of component 2/2' would be modified accordingly. In particular, slot 1b could extend over less than half of the perimeter of bushing 1 and/or could be formed not in the top portion thereof, e.g. if the plugging portions 2b/2c' enter bushing 1 from a side rather than from the top.

## Claims

1. System for the fast connection of a pipe (F) to an outlet (B) and the automatic closing of said outlet (B) in the absence of said pipe (F), **characterized in that** it includes a bushing (1) externally mounted on the outlet (B) and provided with a slot (1b), a gate (2; 2') mounted on the body (C) in which the outlet (B) is formed and mobile between a position closing said bushing (1) through introduction into said slot (1b) and a position opening the bushing (1), and a terminal (3; 3') suitable to be mounted at the end of the pipe (F) and provided with a peripheral groove (3a; 3a') suitable to receive said gate (2; 2'), said terminal (3; 3') being sized and shaped for internal or external coupling with the bushing (1).

2. System according to claim 1, **characterized in that** it further includes a bracket (5) suitable to secure the bushing (1) on the body (C) in which the outlet (B) is formed.

3. System according to claim 1 or 2, **characterized in that** when the terminal (3') is sized and shaped for internal coupling with the bushing (1), said terminal (3') is also provided with a flange (3b') suitable to define its depth of introduction such that when said flange (3b') abuts on the bushing (1) the peripheral groove (3a') is located at the slot (1b).

4. System according to any of the preceding claims, **characterized in that** the movement of the gate (2) between the bushing (1) closing and opening positions is a linear translational movement.

5. System according to claim 4, **characterized in that** the gate (2) is provided with elongated vertical eyelets (2a) to the sides and with a central opening (2c) that is shaped an as inverted keyhole in which the top portion affected by the travel of the gate (2) during the operation thereof has a width smaller than a portion (1c) of the bushing (1), whereas the bottom portion of said central opening (2c) is sufficiently large as to allow the passage of the bushing (1) upon mounting prior to the gate (2) being secured on the body (C).

6. System according to any of claims 1 to 3, **characterized in that** the movement of the gate (2') between the bushing (1) closing and opening positions is a rotational movement.

7. System according to claim 6, **characterized in that** the gate (2') is provided on a side with a hole (2a') for a first means (4) for securing it on the body (C) and on the opposite side with an arcuate slot (2b'), for a second securing means (4), having a radius and a center of curvature that allow the rotation of the gate (2') around said hole (2a').

8. System according to any of the preceding claims, **characterized in that** the bushing (1) is mounted on an outlet (B) by introducing therein a corresponding rear portion (1a) of reduced size with respect to an adjacent portion (1c).

9. System according to any of the preceding claims, **characterized in that** it further includes a return spring biasing the gate (2; 2') towards the closing position.

10. Apparatus for disinfecting medical items, **characterized in that** it includes a trolley provided with at least one system according to any of the preceding claims.

## Patentansprüche

1. System zum schnellen Anschließen eines Rohres (F) an einen Auslass (B) und zum automatischen Verschließen des Auslasses (B) in Abwesenheit des besagten Rohres (F), **dadurch gekennzeichnet, dass** es eine Hülse (1) umfasst, die an dem Auslass (B) extern montiert ist und mit einem Schlitz (1b) versehen ist, ein an dem Körper (C), worin der Auslass (B) eingeformt ist, montiertes Gatter (2; 2'), welches beweglich ist zwischen einer die besagte Hülse (1) durch Einschieben in den besagten Schlitz (1 b) verschließenden Position und einer die Hülse (1) freigebenden Position, und ein Anschlusselement (3; 3'), welches geeignet ist, an dem Ende des Rohrs (F) montiert zu werden, und welches mit einer peripheren Nut (3a; 3a') versehen ist, die geeignet ist, das besagte Gatter (2; 2') aufzunehmen, wobei das besagte Anschlusselement (3; 3') zur internen oder externen Kopplung mit der Hülse (1) dimensioniert und gestaltet ist.

2. System nach Anspruch 1, **dadurch gekennzeichnet, dass** es ferner eine Klammer (5) umfasst, die geeignet ist, die Hülse (1) an dem Körper (C), worin der Auslass (B) eingeformt ist, festzulegen.

3. System nach Anspruch 1 oder 2, **dadurch gekennzeichnet, dass** in dem Fall, wenn das Anschlusselement (3') zur internen Kopplung mit der Hülse (1) dimensioniert und gestaltet ist, das besagte Anschlusselement (3') auch mit einem Flansch (3b') versehen ist, welcher geeignet ist, seine Einschubtiefe derart festzulegen, dass dann, wenn der besagte Flansch (2b') an der Hülse (1) anliegt, die periphere Nut (3a') an dem Schlitz (1 b) platziert ist.

4. System nach einem der vorangehenden Ansprüche, **dadurch gekennzeichnet, dass** die Bewegung des Gatters (2) zwischen den die Hülse (1) schließenden und öffnenden Positionen eine lineare Translationsbewegung ist.

5. System nach Anspruch 4, **dadurch gekennzeichnet, dass** das Gatter (2) an den Seiten mit langgestreckten, vertikalen Ösen (2a) versehen ist sowie mit einer zentralen Öffnung (2c) von der Gestalt eines umgedrehten Schlüssellochs, in welcher der obere, von der Bewegung des Gatters (2) während dessen Betriebs betroffene Bereich eine Breite hat, die kleiner ist als ein Bereich (1c) der Hülse (1), während der untere Bereich der zentralen Öffnung (2c) ausreichend groß ist, um bei der Montage den Durchtritt der Hülse (1) zu erlauben, bevor das Gatter (2) an dem Körper (C) festgelegt wird.

6. System nach einem der Ansprüche 1 bis 3, **dadurch gekennzeichnet, dass** die Bewegung des Gatters (2') zwischen den die Hülse (1) schließenden und öffnenden Positionen eine Rotationsbewegung ist.

7. System nach Anspruch 6, **dadurch gekennzeichnet, dass** das Gatter (2') an einer Seite mit einem Loch (2a') versehen ist für ein erstes Mittel (4) zu seiner Festlegung an dem Körper (C), und an seiner gegenüber liegenden Seite mit einem gebogenen Schlitz (2b'), für ein zweites Befestigungsmittel (4), mit einem Radius und einem Krümmungsmittelpunkt, welche eine Rotation des Gatters (2') um das besagte Loch (2a') erlauben.

8. System nach einem der vorangehenden Ansprüche, **dadurch gekennzeichnet, dass** die Hülse (1) an einem Auslass (B) montiert ist, indem ein rückwärtiger Bereich (1a) mit einer gegenüber einem angrenzenden Bereich (1c) reduzierten Größe dort hineingeschoben wird.

9. System nach einem der vorangehenden Ansprüche, **dadurch gekennzeichnet, dass** es ferner eine Rückholfeder umfasst, welche das Gatter (2; 2') in die Schließposition vorspannt.

10. Vorrichtung zur Desinfektion medizinischer Artikel, **dadurch gekennzeichnet, dass** es einen Rollwagen umfasst, der mit wenigstens einem System nach einem der vorangehenden Ansprüche versehen ist.

## Revendications

1. Système pour le raccordement rapide d'un tuyau (F) à une sortie (B) et la fermeture automatique de ladite sortie (B) en l'absence dudit tuyau (F), **caractérisé en ce qu'**il comprend une douille (1) montée extérieurement sur la sortie (B) et prévue avec une fente (1 b), un opercule (2 ; 2') monté sur le corps (C) dans lequel la sortie (B) est formée et mobile entre une position fermant ladite douille (1) par l'introduction dans ladite fente (1b) et une position ouvrant la douille (1), et une borne (3 ; 3') appropriée pour être montée au niveau de l'extrémité du tuyau (F) et prévue avec une rainure périphérique (3a ; 3a') appropriée pour recevoir ledit opercule (2 ; 2'), ladite borne (3 ; 3') étant dimensionnée et formée pour le couplage interne ou externe avec la douille (1).

2. Système selon la revendication 1, **caractérisé en ce qu'**il comprend en outre un support (5) approprié pour fixer la douille (1) sur le corps (C) dans lequel la sortie (B) est formée.

3. Système selon la revendication 1 ou 2, **caractérisé en ce que** lorsque la borne (3') est dimensionnée et formée pour le couplage interne avec la douille (1), ladite borne (3') est également prévue avec une bride (3b') appropriée pour définir sa profondeur d'introduction de sorte que lorsque ladite bride (3b') vient en butée sur la douille (1), la rainure périphérique (3a') est positionnée au niveau de la fente (1 b).

4. Système selon l'une quelconque des revendications précédentes, **caractérisé en ce que** le mouvement de l'opercule (2) entre les positions de fermeture et d'ouverture de douille (1) est un mouvement de translation linéaire.

5. Système selon la revendication 4, **caractérisé en ce que** l'opercule (2) est prévu avec des oeillets verticaux allongés (2a) sur les côtés et avec une ouverture centrale (2c) qui est formée comme un trou de serrure inversé, dans laquelle la partie supérieure affectée par le déplacement de l'ouverture (2) pendant son fonctionnement, a une largeur inférieure à une partie (1c) de la douille (1), alors que la partie inférieure du ladite ouverture centrale (2c) est suffisamment grande pour permettre le passage de la douille (1) en phase de montage, avant que l'opercule (2) ne soit fixé sur le corps (C).

6. Système selon l'une quelconque des revendications 1 à 3, **caractérisé en ce que** le déplacement de l'opercule (2') entre les positions de fermeture et d'ouverture de douille (1) est un mouvement rotatif.

7. Système selon la revendication 6, **caractérisé en ce que** l'opercule (2') est prévu sur un côté avec un trou (2a') pour un premier moyen (4) pour le fixer sur le corps (C) et sur un côté opposé avec une fente arquée (2b'), pour un second moyen de fixation (4), ayant un rayon et un centre de courbure qui permettent la rotation de l'opercule (2') autour dudit trou (2a').

8. Système selon l'une quelconque des revendications précédentes, **caractérisé en ce que** la douille (1) est montée sur une sortie (B), en introduisant à l'intérieur de cette dernière, une partie arrière (1a) correspondante de taille réduite par rapport à une partie (1 c) adjacente.

9. Système selon l'une quelconque des revendications précédentes, **caractérisé en ce qu'**il comprend en outre un ressort de rappel sollicitant l'opercule (2 ; 2') vers la position de fermeture.

10. Appareil pour désinfecter des articles médicaux, **caractérisé en ce qu'**il comprend un chariot prévu avec au moins un système selon l'une quelconque des revendications précédentes.
